# EUROPEAN PATENT APPLICATION

(11) **EP 2 886 103 A1**
(43) Date of publication of application: **24.06.2015**
(21) Application number: 13199431.1
(22) Date of filing: 23.12.2013
(51) Int. Cl.: A61K 9/00, A61K 9/70, A61K 31/485

(54) **Pharmaceutical orodispersible film comprising buprenorphine particles with a particular size**

(71) Applicant: Hexal AG, 83607 Holzkirchen (DE)
(72) Inventor: Kohr, Thomas, 83607 Holzkirchen (DE); Nink, Jörg, 83607 Holzkirchen (DE); Born, Max, 83607 Holzkirchen (DE)
(74) Representative: Herzog, Fiesser & Partner Patentanwälte PartG mbB

(57) **Abstract**

The present invention relates to a pharmaceutical orodispersible film composition which comprises buprenorphine in the form of particles having a maximum particle diameter Dv90 of at most 20 micrometer, and the process of preparation thereof.

## Description

The present invention relates to a pharmaceutical composition which comprises buprenorphine or a pharmaceutically acceptable salt thereof and to a process for the preparation a pharmaceutical composition which comprises buprenorphine or a pharmaceutically acceptable salt thereof.

### Background

Buprenorphine, (2*S*)-2-[(5*R*,6*R*,7*R*,14*S*)-9alpha-cyclopropylmethyl-4,5-epoxy-6,14-ethano-3-hydroxy-6-methoxymorphinan-7-yl]-3,3-dimethylbutan-2-ol 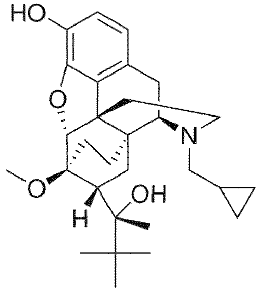 is an opioid agonist which is used to treat opioid addiction such as addiction to heroin and other opiates, and to control pain in non-opioid-tolerant patients. Depending on the application form, buprenorphine is indicated for the treatment of moderate to severe chronic pain or for peri-operative analgesia. Buprenorphine is commonly administered by intramuscular injection, intraveneous infusion, via a transdermal patch, as a sublingual tablet or as an ethanolic liquid oral solution. For sublingual application, two commercially available medications, Subutex® and Suboxone® are known. Subutex® contains only buprenorphine in the form of buprenorphine hydrochloride, whereas Suboxone® contains, in addition to buprenorphine as opioid agonist, naloxone as opioid antagonist.

WO 2011/017483 discloses a film dosage composition which comprises a therapeutically effective amount of buprenorphine or a pharmaceutically acceptable salt thereof, a therapeutically effective amount of naloxone or a pharmaceutically acceptable salt thereof, a polymeric carrier matrix, and a buffer agent. According to WO 2011/017483, these film dosage forms would provide a desired absorption level of both buprenorphine and naloxone, wherein this desired absorption level would be achieved by the buffer agent which should be used in an amount to provide a pH sufficient to optimize the absorption of buprenorphine. In particular with regard to the oral dosage regimen, there is still the need for compositions exhibiting a very good buprenorphine bioavailability.

Thus, it was an object of the present invention to provide a pharmaceutical composition comprising buprenorphine or a pharmaceutically acceptable salt thereof having an improved buprenorphine bioavailability.

It was a further object of the present invention to provide a pharmaceutical composition in the form of an oral dosage composition, comprising buprenorphine or a pharmaceutically acceptable salt thereof having an improved buprenorphine bioavailability.

It was a further object of the present invention to provide a pharmaceutical composition comprising buprenorphine or a pharmaceutically acceptable salt thereof and naloxone or a pharmaceutically acceptable salt thereof, having an improved buprenorphine bioavailability.

It was a further object of the present invention to provide a pharmaceutical composition in the form of an oral dosage composition, comprising buprenorphine or a pharmaceutically acceptable salt thereof and naloxone or a pharmaceutically acceptable salt thereof, having an improved buprenorphine bioavailability.

It was a further object of the present invention to provide a method for the preparation of such pharmaceutical compositions.

### Summary of the Invention

Surprisingly, it was found that these objects can be solved by a pharmaceutical composition comprising buprenorphine or a pharmaceutically acceptable salt thereof wherein the buprenorphine or the pharmaceutically acceptable salt thereof are present as particles exhibiting a specific particle size distribution.

Therefore, the present invention relates to a pharmaceutical composition comprising buprenorphine or a pharmaceutically acceptable salt thereof in the form of particles having a maximum particle diameter Dv90 of at most 20 micrometer.

Further, the present invention relates to a process for preparing a pharmaceutical composition, preferably the pharmaceutical composition, preferably in the form of an oral dosage composition, an injectable dosage composition, or a transdermal dosage composition, more preferably an oral dosage composition, more preferably an oral film dosage composition, said process comprising
(i) providing buprenorphine or a pharmaceutically acceptable salt thereof, preferably in the form of particles having a maximum particle diameter Dv90 of more than 20 micrometer, more preferably at least 50 micrometer, more preferably in the range of from 50 to 150 micrometer, more preferably in the range of from 75 to 125 micrometer;
(ii) dissolving the buprenorphine or the pharmaceutically acceptable salt thereof in a solvent system obtaining a solution;
(iii) preferably subjecting the solution obtained in (ii) to formulation, obtaining a precursor of the pharmaceutical composition comprising at least a portion of the solvent system;
(iv) removing at least a portion, preferably at least 90 weight-%, of the solvent system comprised in the precursor of the pharmaceutical composition obtained in (iii), preferably by drying.

Surprisingly, it was found that compared to the compositions known in the art which contain the buprenorphine or the pharmaceutically acceptable salt thereof with a maximum particle size Dv90 of about 100 micrometer, the pharmaceutical compositions of the present invention exhibit a higher in vivo bioavailability of buprenorphine. Therefore, the pharmaceutical compositions of the present invention allow to achieve the same bioavailability results as the prior art compositions with a decreased content of buprenorphine or, depending on the desired dosage, to achieve better bioavailability results than the prior art compositions with a decreased content of buprenorphine.

The term "buprenorphine or a pharmaceutically acceptable salt thereof in the form of particles" refers to particles wherein at least 95 weight-%, preferably at least 98 weight-%, more preferably at least 99 weight-%, more preferably at least 99.9 weight-%, more preferably at least 99.99 weight-% of a given particle consists of the buprenorphine or the pharmaceutically acceptable salt thereof.

The term "buprenorphine" as used according to the present invention relates the buprenorphine free base, to a metabolite of buprenorphine such as norbuprenorphine, and to a prodrug of buprenorphine. Preferably, the term "buprenorphine" relates to the buprenorphine free base.

### Detailed Description of the Invention

### Pharmaceutical Composition

In particular, the present invention relates to a pharmaceutical composition comprising buprenorphine or a pharmaceutically acceptable salt thereof in the form of particles having a maximum particle diameter Dv90 in the range of from 5 to 20 micrometer, preferably from 5 to 18 micrometer, more preferably from 5 to 16 micrometer, more preferably from 5 to 14 micrometer, more preferably from 5 to 12 micrometer, more preferably from 5 to 10 micrometer.

Therefore, the present invention relates to the pharmaceutical composition described above, wherein the Dv90 is in the range of from 5 to 20 micrometer, preferably from 5 to 10 micrometer.

Generally, the Dv50 of the particles of the buprenorphine or the pharmaceutically acceptable salt thereof comprised in the pharmaceutical composition of the present invention is not subject to specific restrictions. Preferably, the pharmaceutical composition of the present invention comprises the particles of buprenorphine or the pharmaceutically acceptable salt thereof having a maximum particle diameter Dv50 of at most 5 micrometer, more preferably in the range of from 2 to 5 micrometer, more preferably in the range of from 2 to 3 micrometer.

Generally, the Dv10 of the particles of the buprenorphine or the pharmaceutically acceptable salt thereof comprised in the pharmaceutical composition of the present invention is not subject to specific restrictions. Preferably, the pharmaceutical composition of the present invention comprises the particles of buprenorphine or the pharmaceutically acceptable salt thereof having a maximum particle diameter Dv10 0 of at most 1.5 micrometer, more preferably in the range of from 0.5 to 1.5 micrometer, more preferably from 0.5 to 1.2 micrometer.

Preferably, the pharmaceutical composition of the present invention comprises the particles of buprenorphine or the pharmaceutically acceptable salt thereof having a maximum particle diameter Dv50 of at most 5 micrometer, preferably in the range of from 2 to 5 micrometer, more preferably in the range of from 2 to 3 micrometer and a maximum particle diameter Dv10 of at most 1.5 micrometer, preferably in the range of from 0.5 to 1.5 micrometer, more preferably from 0.5 to 1.2 micrometer.

More preferably, the pharmaceutical composition of the present invention comprises the particles of buprenorphine or the pharmaceutically acceptable salt thereof having a maximum particle diameter Dv90 of at most 20 micrometer, preferably in the range of from 5 to 20 micrometer, more preferably in the range of from 5 to 10 micrometer, a maximum particle diameter Dv50 of at most 5 micrometer, preferably in the range of from 2 to 5 micrometer, more preferably in the range of from 2 to 3 micrometer, and a maximum particle diameter Dv10 of at most 1.5 micrometer, preferably in the range of from 0.5 to 1.5 micrometer, more preferably from 0.5 to 1.2 micrometer.

The term "Dv90" as used according to the present invention relates to the maximum particle diameter of the buprenorphine or the pharmaceutically acceptable salt thereof below which 90 % of a sample volume comprising the buprenorphine or the pharmaceutically acceptable salt thereof exists. The term "Dv50" as used according to the present invention relates to the maximum particle diameter of the buprenorphine or the pharmaceutically acceptable salt thereof below which 50 % of a sample volume comprising the buprenorphine or the pharmaceutically acceptable salt thereof exists. The term "Dv10" as used according to the present invention relates to the maximum particle diameter of the buprenorphine or the pharmaceutically acceptable salt thereof below which 10 % of a sample volume comprising the buprenorphine or the pharmaceutically acceptable salt thereof exists. The method by which the Dv90, the Dv50, and the Dv10 are determined according to the present invention is described in detail in Reference Example 1 hereinunder.

Regarding the pharmaceutically acceptable salts of buprenorphine, no specific restrictions exist. Generally, the term "pharmaceutically acceptable salt" refers to a salt which is formed with either an acidic and/or basic portion of buprenorphine. Such salts include, but are not restricted to, the salts as listed, for example, in "Handbook of pharmaceutical salts: Properties, Selection and Use"; P. H. Stahl and C. G. Wermuth (eds.), Wiley-VCH, New York 2002. Preferably, the pharmaceutically acceptable salt of buprenorphine is selected from the group consisting of salts of buprenorphine with a carboxylic acid which is preferably selected from the group consisting of acetic acid, propionic acid, citric acid, tartaric acid, malic acid, maleic acid, lactic acid, malonic acid, fumaric acid, salicylic acid, succinic acid, ascorbic acid, benzoic acid, and a mixtures of two or more thereof, with a hydrohalide preferably selected from hydrochloride and hydrobromide, more preferably hydrochloride, with phosphoric acid, with sulfuric acid, and a mixture of two or more thereof. More preferably, the pharmaceutically acceptable salt of buprenorphine is a buprenorphine hydrohalide, more preferably buprenorphine hydrochloride.

Preferably, the pharmaceutical composition of the present invention additionally contains at least one opioid antagonist or a pharmaceutically acceptable salt thereof wherein the at least one opioid antagonist may include a mu opioid antagonist, a kappa opioid antagonist, or a delta opioid antagonist. More preferably, the at least one antagonist is a mu opioid antagonist. More preferably, the antagonist is selected from the group consisting of levallorphan, naltrexone, nalorphine, and naloxone. More preferably, the opioid antagonist is naloxone. Therefore, the present invention also relates to the pharmaceutical composition described above, additionally comprising at least one opioid antagonist or a pharmaceutically acceptable salt thereof, preferably naloxone or a pharmaceutically acceptable salt thereof. Naloxone, (1S,SR,13R,17S)-10,17-dihydroxy-4-(prop-2-en-1-yl)12-oxa-4-azapentacyclo-[9.6.1.0^{1,11}.0^{5,17}.0^{7,18}]octadeca-7(18)8,10-trien-14-one, has the following formula:

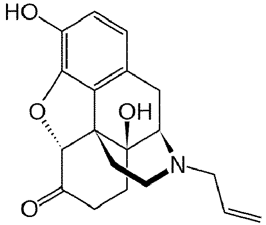

Regarding the pharmaceutically acceptable salts of naloxone, no specific restrictions exist. Generally, the term "pharmaceutically acceptable salt" refers to a salt which is formed with either an acidic and/or basic portion of naloxone. Such salts include, but are not restricted to, the salts as listed, for example, in "Handbook of pharmaceutical salts: Properties, Selection and Use"; P. H. Stahl and C. G. Wermuth (eds.), Wiley-VCH, New York 2002. Preferably, the pharmaceutically acceptable salt of naloxone is selected from the group consisting of salts of naloxone with a carboxylic acid which is preferably selected from the group consisting of acetic acid, propionic acid, citric acid, tartaric acid, malic acid, maleic acid, lactic acid, malonic acid, fumaric acid, salicylic acid, succinic acid, ascorbic acid, benzoic acid, and a mixtures of two or more thereof with a hydrohalide preferably selected from hydrochloride and hydrobromide, more preferably hydrochloride, with phosphoric acid, with sulfuric acid, and a mixture of two or more thereof. More preferably, the pharmaceutically acceptable salt of naloxone is a naloxone hydrohalide, more preferably naloxone hydrochloride.

Therefore, the present invention relates to the pharmaceutical composition described above, comprising buprenorphine or a pharmaceutically acceptable salt thereof, preferably comprising buprenorphine hydrochloride, in the form of particles having a maximum particle diameter Dv90 of at most 20 micrometer, preferably in the range of from 5 to 20 micrometer, more preferably from 5 to 10 micrometer, said pharmaceutical composition additionally comprising naloxone or a pharmaceutically acceptable salt thereof, preferably comprising naloxone hydrochloride.

The at least one opioid antagonist, preferably naloxone, or the pharmaceutically acceptable salt thereof, can be comprised in the pharmaceutical composition of the present invention in an amorphous form or in one or more crystalline forms. If it is comprised in one or more crystalline forms, no specific restrictions exist regarding its particle size. Preferably, in particular in case the at least one opioid antagonist is naloxone or a pharmaceutically acceptable salt thereof, it is comprised in the pharmaceutical composition of the present invention at least partially in an amorphous form. More preferably, at least 99 weight-%, preferably at least 99.5 weight-%, more preferably at least 99.9 weight-% of the at least one opioid antagonist, in particular the naloxone or the pharmaceutically acceptable salt thereof, are present in the amorphous form.

Regarding the pharmaceutical compositions comprising buprenorphine or a pharmaceutically acceptable salt thereof and naloxone or a pharmaceutically acceptable salt thereof, the relative molar amounts of the compounds is not subject to any specific restrictions. Preferably, the pharmaceutical compositions of the present invention, comprising the naloxone or the pharmaceutically acceptable salt thereof, comprise the naloxone relative to buprenorphine at a molar ratio in the range of from 1:1 to 5:1, more preferably from 1:1 to 4:1, more preferably from 1:1 to 3:1.

Preferably, the pharmaceutical composition of the present invention is in the form of an oral dosage composition, an injectable dosage composition such as an intramuscular injection, or a transdermal dosage composition such as in the form of a transdermal patch. More preferably, the pharmaceutical composition of the present invention is in the form of an oral dosage composition such as a tablet or a film. More preferably, the pharmaceutical composition of the present invention is in the form of an oral film dosage composition. More preferably, the pharmaceutical composition of the present invention is in the form of a sublingual film dosage composition.

Therefore, the present invention also relates to the pharmaceutical composition described above in the form of an oral film dosage composition, preferably a sublingual film dosage composition, comprising buprenorphine or a pharmaceutically acceptable salt thereof, preferably comprising buprenorphine hydrochloride, in the form of particles having a maximum particle diameter Dv90 of at most 20 micrometer, preferably in the range of from 5 to 20 micrometer, more preferably from 5 to 10 micrometer, said pharmaceutical composition additionally comprising naloxone or a pharmaceutically acceptable salt thereof, preferably comprising naloxone hydrochloride. Thus, the present invention also relates to an oral film dosage composition, preferably a sublingual film dosage composition, comprising buprenorphine or a pharmaceutically acceptable salt thereof, preferably comprising buprenorphine hydrochloride, in the form of particles having a maximum particle diameter Dv90 of at most 20 micrometer, preferably in the range of from 5 to 20 micrometer, more preferably from 5 to 10 micrometer, said pharmaceutical composition additionally comprising naloxone or a pharmaceutically acceptable salt thereof, preferably comprising naloxone hydrochloride.

Depending on the respective dosage form, the pharmaceutical composition of the present invention may comprise, in addition to the buprenorphine or the pharmaceutically acceptable salt thereof and optionally the at least one opioid antagonist, preferably naloxone, or the pharmaceutically acceptable salt thereof, at least one excipient. Generally, there are no specific restrictions concerning the chemical nature of these excipients provided that the excipient or mixture of excipients comprised in the pharmaceutical composition is/are pharmaceutically acceptable. A pharmaceutically acceptable excipient is any excipient which is relatively non-toxic and innocuous to a patient at concentrations consistent with effective activity of the buprenorphine or the pharmaceutically acceptable salt thereof and optionally the at least one opioid antagonist, preferably naloxone, or the pharmaceutically acceptable salt thereof so that any side effects ascribable to the excipient do not vitiate the beneficial effects of the buprenorphine or the pharmaceutically acceptable salt thereof and optionally the at least one opioid antagonist, preferably naloxone, or the pharmaceutically acceptable salt thereof. Therefore, according to the present invention, excipients are, for example, buffers, disintegrants, binders, lubricants, fillers, plasticizers, surfactants and wetting agents, film-forming agents and coating materials, sweeteners, flavoring agents, and coloring agents such as example pigments. Other excipients known in the field of pharmaceutical compositions may also be used.

Therefore, the present invention relates to the pharmaceutical composition described above, additionally comprising at least one excipient selected from the group consisting of at least one buffer, at least one flavoring agent, at least one coloring agent, at least one plasticizer, at least one sweetener, at least one film forming agent, at least one disintegrant, at least one binder, at least one lubricant, at least one filler, at least one surfactant, at least one wetting agent, at least one coating material, and a mixture of two or more thereof.

Suitable disintegrants according to the present invention include, but are not limited to, carboxymethylcellulose calcium, carboxymethylcellulose sodium, croscarmellose (cross-linked carboxymethylcellulose) sodium, cross-linked polyvinylpyrrolidone, crospovidone (cross-linked povidone, a synthetic cross-linked homopolymer of N-vinyl-2-pyrrolidone), alginic acid, microcrystalline cellulose (such as refined wood pulp derived from alpha cellulose), hydroxypropyl cellulose, low substituted hydroxypropyl cellulose, polacrillin potassium, sodium alginate, sodium starch glycolate, partially hydrolysed starch, sodium carboxymethyl starch, and starch.

Suitable binders according to the present invention include, but are not limited to, hydroxypropyl cellulose, hypromellose (hydroxypropyl methylcellulose, HPMC), microcrystalline cellulose, acacia, alginic acid, carboxymethylcellulose, ethylcellulose, methylcellulose, hydroxyethyl-cellulose, ethylhydroxyethylcellulose, polyvinyl alcohol, polyacrylates, carboxymethylcellulose calcium, carboxymethylcellulose sodium, compressible sugar, ethylcellulose, gelatin, liquid glucose, methylcellulose, polyvinyl pyrrolidone and pre-gelatinized starch.

Suitable lubricants according to the present invention include, but are not limited to, calcium stearate, magnesium stearate, mineral oil, stearic acid, fumaric acid, sodium stearylfumarate, zinc stearate and polyethylene glycol.

Suitable fillers according to the present invention include, but are not limited to, dibasic calcium phosphate, kaolin, microcrystalline cellulose, silicated microcrystalline cellulose, dicalcium phosphate, tricalcium phosphate, magnesium trisilicate, lactose such as example the anhydrous form or the hydrate form such as the monohydrate form, sugars such as dextrose, maltose, saccharose, glucose, fructose or maltodextrin, sugar alcohols such as mannitol, maltitol, sorbitol, xylitol, powdered cellulose, precipitated calcium carbonate, sodium carbonate, sodium phosphate and starch.

Suitable surfactants and wetting agents according to the present invention include, but are not limited to, heptadecaethylene oxycetanol, lecithins, sorbitol monooleate, polyoxyethylene sorbitol monooleate, polyoxyethylene stearate, polyoxyethylen sorbitan monolaurate, benzalkonium chloride, nonoxynol 10, oxtoxynol 9, polysorbates, for example polysorbate 20, polysorbate 40, polysorbate 60 or polysorbate 80, sorbitan monopalmitate, sodium salts of fatty alcoholsulfates such as sodium lauryl sulfate, sodium dodecylsulfate, sodium salts of sulfosuccinates such as sodium dioctylsulfosuccinate, partially esters of fatty acids with alcohols such as glycerine monostearate, partially esters of fatty acids with sorbitans such as sorbitan monolaurate, partially esters of fatty acids with polyhydroxyethylene sorbitans such as polyethyleneglycol sorbitan monolaurate, -monostearate or - monooleate, ethers of fatty alcohols with polyhydroxyethylene, esters of fatty acids with polyhydroxyethylene, copolymers of ethylenoxide and propylenoxide (Pluronic®) and ethoxylated triglycerides.

Suitable buffers according to the present invention include, but are not limited to, pharmaceutically acceptable buffer systems which allow to keep the pH in a range of from 1.5 to 6.5, preferably from 2 to 6. Preferred pH ranges according to the present invention are from 2 to 4 or from 3 to 5 or from 4 to 6. Preferred buffer systems include sodium citrate, citric acid, and combinations thereof.

Suitable film-forming agents and coating materials according to the present invention include, but are not limited to, polyalkylene oxides such as polyethylene oxides, liquid glucose, hydroxyethyl cellulose, hydroxypropyl cellulose, hydroxypropyl methylcellulose (hypromellose, HPMC), methylcellulose, ethylcellulose, cellulose acetate phthalate, shellac, polyvinyl-pyrrolidone, copolymers of vinylpyrrolidone and vinylacetate such as Kollidon® VA64 BASF, copolymers of acrylic and/or methacrylic acid esters with trimethylammoniummethylacrylate, copolymers of dimethylaminomethacrylic acid and neutral methacrylic acid esters, polymers of methacrylic acid or methacrylic acid esters, copolymers of acrylic acid ethylester and methacrylic acid methyl ester, and copolymers of acrylic acid and acrylic acid methylester.

Suitable plasticizers according to the present invention include, but are not limited to, propylene glycol, polyethylene glycol, diethyl phthalate and glycerol.

Suitable coloring agents according to the present invention include, but are not limited to, pigments, inorganic pigments, FD&C Red No. 3, FD&C Red No. 20, FD&C Yellow No. 6, FD&C Blue No. 2, D&C Green No. 5, D&C Orange No. 5, D&C Red No. 8, caramel, ferric oxide red, ferric oxide yellow and titanium dioxide.

Suitable further commonly used excipients which may be used according to the present invention include, but are not limited to, acidifying agents such as acetic acid, citric acid, fumaric acid, hydrochloric acid and nitric acid; alkalizing agents such as ammonia solution, ammonium carbonate, diethanolamine, monoethanolamine, potassium hydroxide, sodium borate, sodium carbonate, sodium hydroxide, triethanolamine and trolamine; adsorbents such as powdered cellulose and activated charcoal; stabilizers and antioxidants such as ascorbic acid, ascorbyl palmitate, butylated hydroxyanisole, butylated hydroxytoluene, hypophosphorus acid, monothioglycerol, propyl gallate, sodium ascorbate, sodium bisulfite, sodium formaldehyde sulfoxylate and sodium metabisulfite; binding materials such as block polymers, natural and synthetic rubber, polyacrylates, polyurethanes, silicones, polysiloxanes and styrene-butadiene copolymers; buffering agents such as potassium metaphosphate, dipotassium phosphate, sodium acetate, sodium citrate anhydrous and sodium citrate hydrates; encapsulating agents such as gelatin, starch and cellulose derivatives; flavorants, masking agents and odors such as anise oil, cinnamon oil, cocoa, menthol, orange oil, peppermint oil and vanillin; humectants such as glycerol, propylene glycol and sorbitol; sweeteners such as aspartame, dextrose, glycerol, mannitol, propylene glycol, saccharin sodium, sorbitol and sucrose; anti-adherents such as magnesium stearate and talc; direct compression excipients such as dibasic calcium phosphate, lactose and microcrystalline cellulose; polishing agents such as carnauba wax and white wax.

Preferably, in particular in case the pharmaceutical composition of the present invention is an oral dosage composition, preferably an oral film dosage composition, the pharmaceutical composition contains at least one buffer, optionally at least one plasticizer, and at least one film forming agent. The relative amounts of the at least one buffer, optionally at least one plasticizer, and at least one film forming agent are not subject to any specific restrictions provided that the desired characteristics of the pharmaceutical composition, preferably of the oral dosage composition, more preferably of the oral film dosage composition are realized. Preferably, the pharmaceutical composition, more preferably the oral dosage composition, more preferably the oral film dosage composition according to the present invention comprises at least one buffer, at least one plasticizer, and at least one film forming agent,
wherein the ratio of the weight of the at least one buffer relative to the sum of the weights of buprenorphine or the pharmaceutically acceptable salt thereof and naloxone or the pharmaceutically acceptable salt thereof is in the range of from 0.25:1 to 0.55:1, preferably from 0.35:1 to 0.45:1;
wherein the ratio of the weight of the at least one plasticizer, if present, relative to the sum of the weights of buprenorphine or the pharmaceutically acceptable salt thereof and naloxone or the pharmaceutically acceptable salt thereof is in the range of from 0.05:1 to 0.3:1, preferably from 0.1:1 to 0.2:1; and
wherein the ratio of the weight of the at least one film forming agent relative to the sum of the weights of buprenorphine or the pharmaceutically acceptable salt thereof and naloxone or the pharmaceutically acceptable salt thereof is in the range of from 1:1 to 5:1, preferably from 2:1 1 to 3:1.

Preferably, the present invention relates to the pharmaceutical composition described above, wherein the at least one buffer is selected from the group consisting of citric acid, sodium citrate, and a mixture thereof, the at least one buffer preferably comprising, more preferably consisting of a mixture of citric acid and sodium citrate;
wherein the at least one plasticizer, if present, is selected from the group consisting of propylene glycol, polyethylene glycol, glycerol, and a mixture thereof, the at least one plasticizer preferably comprising, more preferably consisting of propylene glycol; and
wherein the at least one film forming agent is selected from the group consisting of hydroxyethyl cellulose, hydroxypropyl cellulose, hydroxypropyl methylcellulose, and a mixture of two or more thereof, the at least one film forming agent preferably comprising, more preferably consisting of a mixture of at least one polyethylene oxide and hydroxypropyl methylcellulose.

Therefore, the present invention also relates to the pharmaceutical composition described above, preferably in the form of an oral dosage composition, more preferably of an oral film dosage composition, the pharmaceutical composition comprising buprenorphine or a pharmaceutically acceptable salt thereof, preferably comprising buprenorphine hydrochloride, in the form of particles having a maximum particle diameter Dv90 of at most 20 micrometer, preferably in the range of from 5 to 20 micrometer, more preferably from 5 to 10 micrometer, said pharmaceutical composition optionally additionally comprising naloxone or a pharmaceutically acceptable salt thereof, preferably comprising naloxone hydrochloride, and further comprising at least one buffer, optionally at least one plasticizer, and at least one film forming agent,
wherein the ratio of the weight of the at least one buffer relative to the sum of the weights of buprenorphine or the pharmaceutically acceptable salt thereof and naloxone or the pharmaceutically acceptable salt thereof is in the range of from 0.25:1 to 0.55:1, preferably from 0.35:1 to 0.45:1, wherein the at least one buffer is selected from the group consisting of citric acid, sodium citrate, and a mixture thereof, the at least one buffer preferably comprising, more preferably consisting of a mixture of citric acid and sodium citrate;
wherein the ratio of the weight of the at least one plasticizer, if present, relative to the sum of the weights of buprenorphine or the pharmaceutically acceptable salt thereof and naloxone or the pharmaceutically acceptable salt thereof is in the range of from 0.05:1 to 0.3:1, preferably from 0.1:1 to 0.2:1, wherein the at least one plasticizer is selected from the group consisting of propylene glycol, polyethylene glycol, glycerol, and a mixture thereof, the at least one plasticizer preferably comprising, more preferably consisting of propylene glycol; and
wherein the ratio of the weight of the at least one film forming agent relative to the sum of the weights of buprenorphine or the pharmaceutically acceptable salt thereof and naloxone or the pharmaceutically acceptable salt thereof is in the range of from 1:1 to 5:1, preferably from 2:1 to 3:1, wherein the at least one film forming agent is selected from the group consisting of hydroxyethyl cellulose, hydroxypropyl cellulose, hydroxypropyl methylcellulose, and a mixture of two or more thereof, the at least one film forming agent preferably comprising, more preferably consisting of a mixture of at least one polyethylene oxide and hydroxypropyl methylcellulose.

Preferably, in particular in case the pharmaceutical composition of the present invention is an oral dosage composition, preferably an oral film dosage composition, the pharmaceutical composition contains at least one sweetener, or at least one flavoring agent, or at least one coloring agent, or at least one sweetener and at least one flavoring agent, or at least one sweetener and at least one coloring agent, or at least one flavoring agent and at least one coloring agent, or at least one sweetener and at least one flavoring agent and at least one coloring agent. More preferably, in particular in case the pharmaceutical composition of the present invention is an oral dosage composition, preferably an oral film dosage composition, the pharmaceutical composition contains at least one sweetener and at least one flavoring agent and at least one coloring agent. The relative amounts of the at least one sweetener, at least one flavoring agent, and at least one coloring agent are not subject to any specific restrictions provided that the desired characteristics of the pharmaceutical composition, preferably of the oral dosage composition, more preferably of the oral film dosage composition are realized. Preferably, the pharmaceutical composition, more preferably the oral dosage composition, more preferably the oral film dosage composition according to the present invention comprises at least one sweetener, and at least one flavoring agent, and at least one coloring agent,
wherein the ratio of the weight of the at least sweetener relative to the sum of the weights of buprenorphine or the pharmaceutically acceptable salt thereof and naloxone or the pharmaceutically acceptable salt thereof is in the range of from 0.4:1 to 1:1, preferably from 0.6:1 to 0.7:1;
wherein the ratio of the weight of the at least one flavoring agent relative to the sum of the weights of buprenorphine or the pharmaceutically acceptable salt thereof and naloxone or the pharmaceutically acceptable salt thereof is in the range of from 0.001:1 to 0.01:1, preferably from 0.002:1 to 0.005:1; and
wherein the ratio of the weight of the at least one coloring agent relative to the sum of the weights of buprenorphine or the pharmaceutically acceptable salt thereof and naloxone or the pharmaceutically acceptable salt thereof is in the range of from 0.0002:1 to 0.005:1, preferably from 0.0005:1 to 0.001:1.

Preferably, the present invention relates to the pharmaceutical composition described above, wherein the at least one sweetener is selected from the group consisting of glucose (corn syrup), dextrose, invert sugar, fructose, and combinations thereof; saccharin and its various salts such as the sodium salt; dipeptide sweeteners such as aspartame, dihydrochalcone compounds, glycyrrhizin; Stevia Rebaudiana (Stevioside); chloro derivatives of sucrose such as sucralose; sugar alcohols such as sorbitol, mannitol, xylitol, and the like. Also contemplated are hydrogenated starch hydrolysates and the synthetic sweetener 3,6-dihydro-6-methyl-1-1-1,2,3-oxathiazin-4-one-2,2-dioxide, particularly the potassium salt thereof (acesulfame-K), and sodium and calcium salts thereof, natural intensive sweeteners, and a mixture thereof;
wherein the at least one flavoring agent is selected from the group consisting of natural and synthetic flavoring liquids, such as lemon, orange, grape, lime and grapefruit and fruit essences including apple, pear, peach, grape, strawberry, raspberry, cherry, plum, pineapple, apricot or other fruit flavors, aldehydes and esters such as benzaldehyde (cherry, almond), citral i.e., alphacitral (lemon, lime), neral, i.e., beta-citral (lemon, lime), decanal (orange, lemon), aldehyde C-8 (citrus fruits), aldehyde C-9 (citrus fruits), aldehyde C- 12 (citrus fruits), to IyI aldehyde (cherry, almond), 2,6-dimethyloctanol (green fruit), and 2-dodecenal (citrus, mandarin), combinations thereof and a mixture thereof; and
wherein the at least one coloring agent is selected from the group consisting of food, drug and cosmetic colors (FD&C), drug and cosmetic colors such as dyes, their corresponding lakes, natural and derived colorants, azo dyes, organic or inorganic pigments such as the oxides or iron or titanium, or coloring agents of natural origin" and a mixture of two or more thereof.

As mentioned above, the pharmaceutical compositions according to the present invention comprising buprenorphine or a pharmaceutically acceptable salt thereof, preferably comprising buprenorphine hydrochloride, in the form of particles having a maximum particle diameter Dv90 of at most 20 micrometer, preferably in the range of from 5 to 20 micrometer, more preferably from 5 to 10 micrometer, exhibit a higher *in vivo* bioavailability of buprenorphine than the compositions of the prior art which contain the buprenorphine or the pharmaceutically acceptable salt thereof in the form of particles having a maximum particle diameter Dv90 of about 90 micrometer. The term *"in vivo* bioavailability of buprenorphine" as used in this context is to be understood as being determined as described in Reference Example 2 hereinunder. Preferably, the buprenorphine *in vivo* bioavailability of the pharmaceutical compositions of the present invention at least 10 %, more preferably at least 20 % higher than the buprenorphine *in vivo* bioavailability of the pharmaceutical compositions of the prior art, wherein the buprenorphine *in vivo* bioavailability of the pharmaceutical compositions of the present invention is preferably of from 20 to 50 % higher than the buprenorphine *in vivo* bioavailability of the pharmaceutical compositions of the prior art. Therefore, the present invention also relates to the pharmaceutical composition described above, preferably the oral dosage composition, more preferably the oral film dosage composition according to the present invention, having a buprenorphine *in vivo* bioavailability ratio of at least 1.1:1, preferably at least 1.2:1, more preferably in the range of from 1.2:1 to 1.5:1, wherein the buprenorphine in vivo bioavailability ratio is defined as the buprenorphine in vivo bioavailability of the pharmaceutical composition of the present invention relative to the buprenorphine in vivo bioavailability of an otherwise identical composition comprising buprenorphine or a pharmaceutically acceptable salt thereof in the form of particles having a maximum particle diameter Dv90 of (100 ± 10) micrometer. The bioavailability is determined using the parameters AUCₜ, AUC_{inf}, and cₘₐₓ according to standard procedures known in the art and exemplified in Reference Example 2 of the present application. Regardless which of these parameters is used for comparing the inventive pharmaceutical composition with the compositions having a Dv90 of (100 ± 10) micrometer, the bioavailability ratio is in the above-mentioned preferred ranges.

Generally, the present invention further relates to particles of buprenorphine or a pharmaceutically acceptable salt thereof, preferably buprenorphine hydrochloride, having a maximum particle diameter Dv90 of at most 20 micrometer, preferably in the range of from 5 to 20 micrometer, more preferably from 5 to 18 micrometer, more preferably from 5 to 16 micrometer, more preferably from 5 to 14 micrometer, more preferably from 5 to 12 micrometer, more preferably from 5 to 10 micrometer. Further, the present invention relates to the use of these particles for the preparation of a pharmaceutical composition, preferably an oral dosage composition, more preferably an oral film dosage composition.

### Preparation process

According to the present invention, it is preferred to prepare the pharmaceutical composition comprising buprenorphine or a pharmaceutically acceptable salt thereof in the form of particles having a maximum particle diameter Dv90 of at most 20 micrometer by a process which comprises dissolving a suitably provided buprenorphine or a pharmaceutically acceptable salt thereof in a suitable solvent system and suitably crystallizing the buprenorphine or the pharmaceutically acceptable salt thereof from the thus obtained solution, thereby obtaining the buprenorphine or the pharmaceutically acceptable salt thereof in the form of the particles having a maximum particle diameter Dv90 of at most 20 micrometer. The term "solvent system" as used in this context of the present invention relates to one single solvent or a mixture of two or more solvents wherein preferably at least 99 weight-%, more preferably at least 99.5 weight-%, more preferably at least 99.9 weight-% of the provided buprenorphine or the pharmaceutically acceptable salt thereof can be dissolved in the solvent system, preferably at ambient conditions.

### Preparation of Particles

Generally, it is conceivable to provide buprenorphine or a pharmaceutically acceptable salt thereof, dissolve the buprenorphine or the pharmaceutically acceptable salt thereof in a suitable solvent system, crystallize the buprenorphine or the pharmaceutically acceptable salt thereof from the thus obtained solution to obtain the buprenorphine or the pharmaceutically acceptable salt thereof in the form of the particles having a maximum particle diameter Dv90 of at most 20 micrometer, and subject the thus obtained particles, optionally after separation from the suspension obtain from the crystallization, to prepare the pharmaceutical composition, wherein said preparation of the pharmaceutical composition may comprise the addition of one or more pharmaceutically acceptable excipients and/ or one or more opioid antagonists, preferably naloxone, or the pharmaceutically acceptable salt thereof. Preferably, the crystallization of the buprenorphine or the pharmaceutically acceptable salt thereof is carried out by suitably removing at least a portion the solvent system from the solution, wherein preferably at least 80 weight-%, more preferable at least 85 weight-%, more preferably at least 90 weight-% of the solvent system are removed. Preferably, said removal of the solvent system is carried out by a suitable drying process. Preferably, the solvent system comprises acetone, ethanol, or a mixture thereof, and optionally additionally water. More preferably, the solvent system comprises acetone and optionally additionally water. More preferably, the solvent system consists of acetone wherein the term "consists of" as used in this context of the present invention relates to a solvent system which, in addition to acetone, may only comprise impurities which are contained in the acetone. Preferred drying processes comprise drying temperatures in the range of from 30 to 110 °C, preferably from 35 to 95 °C, more preferably from 40 to 70 °C, wherein the drying can be accomplished either in batch, in semi-continuous, or in continuous mode, and wherein the drying can be accomplished in one, two, or more different drying temperatures, preferably within the above-mentioned preferred ranges.

Therefore, the present invention also relates to particles of buprenorphine or a pharmaceutically acceptable salt thereof, preferably buprenorphine hydrochloride, having a maximum particle diameter Dv90 of at most 20 micrometer, preferably in the range of from 5 to 20 micrometer, more preferably from 5 to 10 micrometer, wherein the particles of the buprenorphine or the pharmaceutically acceptable salt thereof preferably have a maximum particle diameter Dv50 of at most 5 micrometer, more preferably in the range of from 2 to 5 micrometer, more preferably in the range of from 2 to 3 micrometer, and preferably have a maximum particle diameter Dv10 of at most 1.5 micrometer, more preferably in the range of from 0.5 to 1.5 micrometer, more preferably from 0.5 to 1.2 micrometer.

As mentioned above, these particles are preferably prepared by a process which comprises
(I) providing buprenorphine or a pharmaceutically acceptable salt thereof;
(II) dissolving the buprenorphine or the pharmaceutically acceptable salt thereof in a solvent system obtaining a solution;
(III) removing at least a portion, preferably at least 90 weight-%, of the solvent system, preferably by drying.

Further, the present invention relates to particles of buprenorphine or the pharmaceutically acceptable salt thereof, obtainable or obtained by a process which comprises
(I) providing buprenorphine or a pharmaceutically acceptable salt thereof;
(II) dissolving the buprenorphine or the pharmaceutically acceptable salt thereof in a solvent system obtaining a solution;
(III) removing at least a portion, preferably at least 90 weight-%, of the solvent system, preferably by drying.

Preferably, the buprenorphine or the pharmaceutically acceptable salt thereof is provided in (I) in the form of particles having a maximum particle diameter Dv90 of more than 20 micrometer, more preferably at least 50 micrometer, more preferably in the range of from 50 to 150 micrometer, more preferably in the range of from 75 to 125 micrometer. More preferably, the particles of the buprenorphine or the pharmaceutically acceptable salt thereof provided in (I) have a maximum particle diameter Dv50 of at most 40 micrometer, preferably in the range of from 20 to 60 micrometer, more preferably in the range of from 30 to 50 micrometer, and a maximum particle diameter Dv10 of at most 10 micrometer, preferably in the range of from 6 to 10 micrometer, more preferably from 7 to 9 micrometer. Such particles are commercially available, for example by Noramco Inc.

Therefore, the present invention also relates to the process described above, comprising
(I) providing buprenorphine or a pharmaceutically acceptable salt thereof in the form of particles having a maximum particle diameter Dv90 of more than 20 micrometer, more preferably at least 50 micrometer, more preferably in the range of from 50 to 150 micrometer, more preferably in the range of from 75 to 125 micrometer;
(II) dissolving the buprenorphine or the pharmaceutically acceptable salt thereof in a solvent system obtaining a solution, wherein the solvent system comprises, preferably consists of acetone;
(III) removing at least a portion, preferably at least 90 weight-%, of the solvent system by drying the solution obtained in (II) at a temperature in the range of from 30 to 110 °C, preferably from 35 to 95 °C, more preferably from 40 to 70 °C.

Further, the present invention relates to particles of buprenorphine or the pharmaceutically acceptable salt thereof, obtainable or obtained by a process which comprises
(I) providing buprenorphine or a pharmaceutically acceptable salt thereof in the form of particles having a maximum particle diameter Dv90 of more than 20 micrometer, more preferably at least 50 micrometer, more preferably in the range of from 50 to 150 micrometer, more preferably in the range of from 75 to 125 micrometer;
(II) dissolving the buprenorphine or the pharmaceutically acceptable salt thereof in a solvent system obtaining a solution, wherein the solvent system comprises, preferably consists of acetone;
(III) removing at least a portion, preferably at least 90 weight-%, of the solvent system by drying the solution obtained in (II) at a temperature in the range of from 30 to 110 °C, preferably from 35 to 95 °C, more preferably from 40 to 70 °C.

Further, the present invention relates to the use of the particles of buprenorphine or a pharmaceutically acceptable salt thereof having a maximum particle diameter Dv90 of at most 20 micrometer, preferably in the range of from 5 to 20 micrometer, more preferably from 5 to 10 micrometer, wherein the particles of the buprenorphine or the pharmaceutically acceptable salt thereof preferably have a maximum particle diameter Dv50 of at most 5 micrometer, more preferably in the range of from 2 to 5 micrometer, more preferably in the range of from 2 to 3 micrometer, and preferably have a maximum particle diameter Dv10 of at most 1.5 micrometer, more preferably in the range of from 0.5 to 1.5 micrometer, more preferably from 0.5 to 1.2 micrometer, and of the particles obtainable or obtained by a process comprising
(I) providing buprenorphine or a pharmaceutically acceptable salt thereof;
(II) dissolving the buprenorphine or the pharmaceutically acceptable salt thereof in a solvent system obtaining a solution;
(III) removing at least a portion, preferably at least 90 weight-%, of the solvent system, preferably by drying,
said process preferably comprising
(I) providing buprenorphine or a pharmaceutically acceptable salt thereof in the form of particles having a maximum particle diameter Dv90 of more than 20 micrometer, more preferably at least 50 micrometer, more preferably in the range of from 50 to 150 micrometer, more preferably in the range of from 75 to 125 micrometer;
(II) dissolving the buprenorphine or the pharmaceutically acceptable salt thereof in a solvent system obtaining a solution, wherein the solvent system comprises, preferably consists of acetone;
(III) removing at least a portion, preferably at least 90 weight-%, of the solvent system by drying the solution obtained in (II) at a temperature in the range of from 30 to 110 °C, preferably from 35 to 95 °C, more preferably from 40 to 70 °C,
for the preparation of a pharmaceutical composition, preferably an oral dosage composition, more preferably an oral film dosage composition.

### Preparation of a Pharmaceutical Composition

Preferably according to the present invention, prior to crystallizing the buprenorphine or the pharmaceutically acceptable salt thereof to obtain the particles having a maximum particle diameter Dv90 of at most 20 micrometer, the solution which is obtained by dissolving the suitably provided buprenorphine or the pharmaceutically acceptable salt thereof in the suitable solvent system, is subjected to formulation. From this formulation wherein at least one suitable excipient is added to said solution, a precursor of the pharmaceutical composition is obtained which, in a subsequent step, is subjected to at least partial removal of the solvent system which removal is preferably carried out by drying.

Therefore, the present invention also relates to a process for preparing a pharmaceutical composition, preferably the pharmaceutical composition as described in the section "The Pharmaceutical Composition" hereinabove, preferably in the form of an oral dosage composition, an injectable dosage composition, or a transdermal dosage composition, more preferably an oral dosage composition, more preferably an oral film dosage composition, said process comprising
(i) providing buprenorphine or a pharmaceutically acceptable salt thereof;
(ii) dissolving the buprenorphine or the pharmaceutically acceptable salt thereof in a solvent system obtaining a solution;
(iii) subjecting the solution obtained in (ii) to formulation, obtaining a precursor of the pharmaceutical composition comprising at least a portion of the solvent system, preferably at least 95 weight-%, more preferably at least 98 weight-%, more preferably at least 99 weight-% of the solvent system;
(iv) removing at least a portion, preferably at least 90 weight-%, of the solvent system comprised in the precursor of the pharmaceutical composition obtained in (iii),
Also, the present invention relates to a pharmaceutical composition obtainable or obtained by this process.

Preferably, the buprenorphine or the pharmaceutically acceptable salt thereof is provided in (i) in the form of particles having a maximum particle diameter Dv90 of more than 20 micrometer, more preferably at least 50 micrometer, more preferably in the range of from 50 to 150 micrometer, more preferably in the range of from 75 to 125 micrometer. More preferably, the particles of the buprenorphine or the pharmaceutically acceptable salt thereof provided in (i) have a maximum particle diameter Dv50 of at most 40 micrometer, preferably in the range of from 20 to 60 micrometer, more preferably in the range of from 30 to 50 micrometer, and a maximum particle diameter Dv10 of at most 10 micrometer, preferably in the range of from 6 to 10 micrometer, more preferably from 7 to 9 micrometer. Such particles are commercially available, for example by Noramco Inc.

Preferably, the solvent system comprises acetone, ethanol, or a mixture thereof, and optionally additionally water. More preferably, the solvent system comprises acetone and optionally additionally water. More preferably, the solvent system consists of acetone wherein the term "consists of" as used in this context of the present invention relates to a solvent system which, in addition to acetone, may only comprise impurities which are contained in the acetone.

Generally, the temperature at which the dissolving in (ii) is carried out is not subject to any specific restrictions and will depend on the specific composition of the solvent system. Preferably, the dissolving in (ii) is carried out at a temperature of the solvent system in the range of from 15 to 30 °C, more preferably from 18 to 25 °C, more preferably from 20 to 22 °C.

Preferably, by suitably removing at least a portion the solvent system from the solution according to (iv), the buprenorphine or the pharmaceutically acceptable salt thereof is crystallized, wherein said removal of the solvent system is preferably carried out by a suitable drying process. Preferably, at least 80 weight-%, more preferable at least 85 weight-%, more preferably at least 90 weight-% of the solvent system are removed. Preferably, at least 90 weight-%, more preferably at least 95 weight-%, more preferably at least 98 weight-%, more preferably at least 99 weight-% of the buprenorphine or the pharmaceutically acceptable salt thereof are crystallized in (iv). Therefore, the present invention also relates to the process as described above, comprising
(iv) crystallizing at least 95 weight-%, preferably at least 98 weight-%, more preferably at least 99 weight-% of the buprenorphine or the pharmaceutically acceptable salt thereof comprised dissolved in the precursor of the pharmaceutical composition obtained in (iii), by removing at least a portion, preferably at least 90 weight-%, of the solvent system comprised in the precursor of the pharmaceutical composition obtained in (iii),
Also, the present invention relates to a pharmaceutical composition obtainable or obtained by this process.

Preferred drying processes comprise drying temperatures in the range of from 30 to 110 °C, preferably from 35 to 95 °C, more preferably from 40 to 70 °C, wherein the drying can be accomplished either in batch, in semi-continuous, or in continuous mode, and wherein the drying can be accomplished in one, two, or more different drying temperatures, preferably within the above-mentioned preferred ranges.

Preferably, the drying is carried out semi-continuously or continuously, more preferably continuously. A preferred drying apparatus may include a continuous belt drying tunnel having multiple drying zones located within. The conditions of each drying zone may vary, for example, temperature and humidity may be selectively chosen. It may be desirable to sequentially order the zones to provide a stepped up drying effect.

Preferably, the drying is carried out at two or more temperatures such as at two different temperatures or three different temperatures. Preferably, each of the different temperatures is within the above-mentioned preferred ranges. More preferably, the drying is commenced at a first specific drying temperature for a specific period of time and the continued at a second drying temperature for a specific period of time, optionally followed by a drying at a third specific drying temperature for a specific period of time, wherein the second drying temperature is preferably higher than the first drying temperature and the third drying temperature is preferably higher than the second drying temperature. If, for example, the drying is carried out at two different drying temperature, the first drying temperature is preferably in the range of from 30 to 55 °C, more preferably from 35 to 55 °C, more preferably from 40 to 55 °C, and the second drying temperature is preferably in the range of from 55 to 110 °C, more preferably from 55 to 95 °C, more preferably from 55 to 70 °C. If, for example, the drying is carried out at three different drying temperature, the first drying temperature is preferably in the range of from 30 to 60 °C, more preferably from 35 to 55 °C, more preferably from 40 to 50 °C, the second drying temperature is preferably in the range of from 60 to 90 °C, more preferably from 55 to 75 °C, more preferably from 50 to 60 °C, and the third drying temperature is preferably in the range of from 90 to 110 °C, more preferably from 75 to 95 °C, more preferably from 60 to 70 °C.

Preferably, the drying is carried out in an oxygen containing atmosphere such as air or lean air, or in a nitrogen atmosphere such as technical nitrogen. More preferably, the drying is carried out in an oxygen containing atmosphere such as air or lean air, more preferably air.

Preferably, the drying is carried out for a period of time in the range of from 3 to 30 minutes, more preferably from 5 to 30 minutes, more preferably from 10 to 30 minutes, more preferably from 10 to 25 minutes, more preferably from 10 to 20 minutes. In case the drying is carried out at two or more different drying temperatures, the term "period of time" as used in this context of the present invention relates to the overall drying period of time.

Therefore, the present invention also relates to a process for preparing a pharmaceutical composition, preferably the pharmaceutical composition as described in the section "The Pharmaceutical Composition" hereinabove, preferably in the form of an oral dosage composition, an injectable dosage composition, or a transdermal dosage composition, more preferably an oral dosage composition, more preferably an oral film dosage composition, said process comprising
(i) providing buprenorphine or a pharmaceutically acceptable salt thereof in the form of particles having a maximum particle diameter Dv90 of more than 20 micrometer, more preferably at least 50 micrometer, more preferably in the range of from 50 to 150 micrometer, more preferably in the range of from 75 to 125 micrometer;
(ii) dissolving the buprenorphine or the pharmaceutically acceptable salt thereof in a solvent system obtaining a solution wherein the solvent system comprises, preferably consists of acetone, wherein the dissolving is preferably carried out at a temperature of the solvent system in the range of from 15 to 30 °C, more preferably from 18 to 25 °C, more preferably from 20 to 22 °C;
(iii) subjecting the solution obtained in (ii) to formulation, obtaining a precursor of the pharmaceutical composition comprising at least a portion of the solvent system;
(iv) removing at least a portion, preferably at least 90 weight-%, of the solvent system comprised in the precursor of the pharmaceutical composition obtained in (iii) by drying, preferably at one or
more temperatures in the range of from 30 to 110 °C, more preferably from 35 to 95 °C, more preferably from 40 to 70 °C.
Also, the present invention relates to a pharmaceutical composition obtainable or obtained by this process.

As described above, it is preferred that the pharmaceutical composition of the present invention comprises, in addition to the buprenorphine or the pharmaceutically acceptable salt thereof, at least one opioid antagonist, preferably naloxone, or a pharmaceutically acceptable salt thereof. This at least one opioid antagonist, preferably the naloxone, or the pharmaceutically acceptable salt thereof, generally can be added in every suitable preparation stage. Preferably, the at least one opioid antagonist, preferably the naloxone, or the pharmaceutically acceptable salt thereof is comprised in the solution which is obtained from (ii). Thus, it is preferred to dissolve the buprenorphine or the pharmaceutically acceptable salt thereof and the at least one opioid antagonist, preferably the naloxone, or the pharmaceutically acceptable salt thereof simultaneously or sequentially in the solvent system. It is also possible to dissolve the buprenorphine or the pharmaceutically acceptable salt thereof in a portion of the solvent system, to dissolve the at least one opioid antagonist, preferably the naloxone, or the pharmaceutically acceptable salt thereof in another portion of the solvent system, and to combine the two solutions. In the solution obtained in (ii), preferably at least 95 weight-%, more preferable at least 98 weight-%, more preferably at least 99 weight-% of the at least one opioid antagonist, preferably the naloxone, or the pharmaceutically acceptable salt thereof, are present in the dissolved state.

Preferably, the at least one opioid antagonist, preferably the naloxone, or the pharmaceutically acceptable salt thereof, is employed in the process of the present invention in amount so that the solution obtained in (ii) comprises the dissolved buprenorphine or the pharmaceutically acceptable salt thereof at a molar ratio of the at least one opioid antagonist, preferably naloxone, relative to the buprenorphine in the range of from 1:1 to 5:1, more preferably from 1:1 to 4:1, more preferably from 1:1 to 3:1. According to the present invention, it was found that after step (iv) of the process, at least 99 weight-%, more preferably at least 99.5 weight-%, more preferably at least 99.9 weight-% of the naloxone or the pharmaceutically acceptable salt thereof, preferably the naloxone hydrochloride, are present in the amorphous form.

According to step (iii) of the process of the present invention, the solution obtained in (ii) is subjected to formulation wherefrom a precursor of the pharmaceutical composition comprising at least a portion of the solvent system is obtained. During said subjecting to formulation, at least one pharmaceutically acceptable excipient or a precursor thereof is added to the solution obtained in (ii) wherein this at least one excipient will be contained in the finally obtained pharmaceutical composition. Preferably, according to the present invention, all excipients which are comprised in the finally obtained pharmaceutical composition are added, either as such or in the form of suitable precursor, to the solution obtained in (ii) during subjecting to formulation in (iii). The term "precursor of an excipient" as used in the context of the present invention relates to a compound which is chemically transformed during either (iii), and/or (iv), or any subsequent step to result in the actual excipient.

Therefore, according to the present invention, a process is provided according to which the crystallization of the buprenorphine or the pharmaceutically acceptable salt thereof resulting in particles having a maximum particle diameter Dv90 of at most 20 micrometer, achieved by removal of at least a portion of the solvent system according to (iv), preferably by drying, takes place in a mixture which, in addition to dissolved buprenorphine or the pharmaceutically acceptable salt thereof, contains all excipients or precursors thereof to be contained in the final pharmaceutical composition, and preferably also the at least one opioid antagonist, preferably the naloxone, or the pharmaceutically acceptable salt thereof.

Regarding the chemical nature of preferred excipients and preferred amounts of preferred excipients which are employed according to the process of the present invention, reference is made to the respective description in the section "The Pharmaceutical Composition" hereinabove. Therefore, the present invention also relates to the process as described above, wherein the subjecting to formulation according to (ii) comprises
(iii.1) adding at least one excipient, preferably selected from the group consisting of at least one buffer, at least one flavoring agent, at least one coloring agent, at least one plasticizer, at least one sweetener, at least one film forming agent, at least one disintegrant, at least one binder, at least one lubricant, at least one filler, at least one surfactant, at least one wetting agent, at least one coating material, and a mixture of two or more thereof, , more preferably selected from the group consisting of at least one buffer, at least one plasticizer, at least one film forming agent, at least one sweetener, at least one flavoring agent, at least one coloring agent, and a mixture of two or more thereof, to the solution obtained in (ii), obtaining a mixture comprising the buprenorphine or the pharmaceutically acceptable salt thereof, the at least one excipient and optionally the naloxone or the pharmaceutically acceptable salt thereof, wherein the at least one excipient is preferably added in the form of a solution or a suspension.

Regarding the solvent or mixture of solvents in which the at least one excipient is preferably employed, no specific restrictions exist. Most preferably, a solvent or mixture of solvents is used which can be removed or essentially removed in step (iv) of the process of the invention. Preferably, the solvent or mixture of solvents is selected from the group consisting of water, acetone, ethanol, and a mixture of two or three thereof. More preferably, the solvent or mixture of solvents comprises water, wherein the solvent optionally consists of water wherein the term "consists of" as used in this context of the present invention relates to a solvent which, in addition to water, may only comprise impurities which are contained in the water. Preferably, during (iv), at least 80 weight-%, more preferably at least 85 weight-%, more preferably at least 90 weight-% of the solvent or mixture of solvents in which the at least one excipient is preferably employed, are removed, preferably by drying.

According to a preferred embodiment of the present invention, the pharmaceutical composition is an oral film dosage composition. Preferably, such oral film dosage composition is prepared by subjecting the mixture obtained in (iii.1) to a film forming stage (iii.2).

Generally, it is conceivable that in such film forming stage (iii.2), the mixture obtained in (iii.1) is coated on a suitable film carrier matrix. The chemical composition of the film carrier matrix generally can be chosen according to the specific needs of the pharmaceutical composition provided that it is orally dissolvable. For example, the chemical composition of the film carrier matrix can be designed to allow the preparation of a fast dissolving, a moderately dissolving, or a slowly dissolving pharmaceutical composition wherein fast dissolving films dissolve in about 1 second to about 30 seconds in the mouth, moderately dissolving films dissolve in more than about 30 seconds to about 30 minutes in the mouth, and slowly dissolving films dissolve in more than 30 minutes to about 30 seconds.

Preferably, according to the present invention, the film forming stage (iii.2) comprises forming a wet film from the mixture obtained in (iii.1). Therefore, the present invention also relates to the process described above, further comprising
(iii.2) forming a film, preferably a wet film, from the mixture obtained in (iii.1).

Regarding the method of forming a wet film, no specific restrictions exist. Preferably, in order to prevent the formation of bubbles such as air bubbles in the film of the present invention, the film formation step can be performed under vacuum. Generally, the incorporation of a suitable foam reducing agent into the mixture of (ii) can reduce or eliminate the formation of air bubbles within the film. Generally, mixture obtained in (ii) is formed into a wet film by any method known in the art such as extrusion, coating, spreading, casting or drawing the matrix. If a multi-layered film is desired, this may be accomplished by co-extruding more than one combination of components which may be of the same or different composition. A multi-layered wet film may also be achieved by coating, spreading, or casting a matrix onto an already formed film layer.

Although a variety of different film-forming techniques may be used, it is desirable to select a method that will provide a flexible film, such as reverse roll coating. The flexibility of the film allows for the sheets of film to be rolled and transported for storage or prior to being cut/divided into individual dosage forms. Desirably, the film will also be self-supporting or in other words able to maintain its integrity and structure in the absence of a separate support.

Coating or casting methods are particularly useful for the purpose of forming the films of the present invention. Specific examples include reverse roll coating, gravure coating, immersion or dip coating, metering rod or Meyer Bar coating, slot die or extrusion coating, gap or knife over roll coating, air knife coating, curtain coating, or combinations thereof.

Roll coating, or more specifically reverse roll coating, is particularly suitable. This procedure provides excellent control and uniformity of the resulting films. In this procedure, the coating material is measured onto the applicator roller by the precision setting of the gap between the upper metering roller and the application roller below it. The coating is transferred from the application roller to the substrate as it passes around the support roller adjacent to the application roller. Both three roll and four roll processes are common.

The gravure coating process relies on an engraved roller running in a coating bath, which fills the engraved dots or lines of the roller with the coating material. The excess coating on the roller is wiped off by a doctor blade and the coating is then deposited onto the substrate as it passes between the engraved roller and a pressure roller. Offset gravure is common, where the coating is deposited on an intermediate roller before transfer to the substrate.

In the metering rod coating process, an excess of the coating is deposited onto the substrate as it passes over the bath roller. The wire-wound metering rod, sometimes known as a Meyer Bar, allows the desired quantity of the coating to remain on the substrate. The quantity is determined by the diameter of the wire used on the rod.

In the slot die process, the coating is squeezed out by gravity or under pressure through a slot and onto the substrate. If the coating is solid, the process is termed "extrusion" and in this case, the line speed is frequently much faster than the speed of the extrusion. This enables coatings to be considerably thinner than the width of the slot.

It may be particularly desirable to employ extrusion methods for forming the films according to the present invention comprising PEO (polyethylene oxide) polymer components.

The gap or knife over roll process relies on a coating being applied to the substrate which then passes through a "gap" between a "knife" and a support roller. As the coating and substrate pass through, the excess is scraped off. Air knife coating is where the coating is applied to the substrate and the excess is "blown off" by a powerful jet from the air knife. This procedure is useful for aqueous coatings. In the curtain coating process, a bath with a slot in the base allows a continuous curtain of the coating to fall into the gap between two conveyors. The object to be coated is passed along the conveyor at a controlled speed and so receives the coating on its upper face.

Preferred embodiments and combinations of embodiments as indicated by the respective dependencies and back-references of the film forming method preferably applied in stage (iii.2) of the process of the present invention are given in the list of embodiments hereinbelow.

### Uses of the Pharmaceutical Composition

The pharmaceutical compositions of the present invention and/or the pharmaceutical compositions obtainable or obtained according to a process of the present invention are preferably used in a method for treating, reducing, or preventing pain in a mammal, preferably a human. Further, the present invention relates to the use of the pharmaceutical compositions of the present invention and/or the pharmaceutical compositions obtainable or obtained according to a process of the present invention for treating, reducing, or preventing pain in a mammal, preferably a human. Further, the present invention relates to the use of the pharmaceutical compositions of the present invention and/or the pharmaceutical compositions obtainable or obtained according to a process of the present invention for the preparation of a medicament for treating, reducing, or preventing pain in a mammal, preferably a human. Further, the present invention relates to a method for treating, reducing, or preventing pain in a mammal, preferably a human, comprising administering the pharmaceutical composition of the present invention and/or the pharmaceutical compositions obtainable or obtained according to a process of the present invention to a mammalian, preferably human patient in need thereof.

Also, the present invention relates to the pharmaceutical composition of the present invention and/or the pharmaceutical compositions obtainable or obtained according to a process of the present invention for use in a drug substitution therapy of a mammal, preferably a human, wherein the drug is preferably a narcotic more preferably heroine or morphine. Further, the present invention relates to the use of the pharmaceutical compositions of the present invention and/or the pharmaceutical compositions obtainable or obtained according to a process of the present invention in a drug substitution therapy of a mammal, preferably a human. Further, the present invention relates to the pharmaceutical compositions of the present invention and/or the pharmaceutical compositions obtainable or obtained according to a process of the present invention for the preparation of a medicament for a drug substitution therapy of a mammal, preferably a human. Further, the present invention relates to a drug substitution therapy method comprising administering the pharmaceutical compositions of the present invention and/or the pharmaceutical compositions obtainable or obtained according to a process of the present invention to a mammalian, preferably a human patient in need thereof.

Generally, the present invention is illustrated by the following embodiments and combinations of embodiments as indicated by the respective dependencies and back-references:
1. A pharmaceutical composition comprising buprenorphine or a pharmaceutically acceptable salt thereof in the form of particles having a maximum particle diameter Dv90 of at most 20 micrometer.
2. The pharmaceutical composition of embodiment 1, wherein the Dv90 is in the range of from 5 to 20 micrometer, preferably from 5 to 10 micrometer.
3. The pharmaceutical composition of embodiment 1 or 2, wherein the particles of buprenorphine or the pharmaceutically acceptable salt thereof have a maximum particle diameter Dv50 of at most 5 micrometer, preferably in the range of from 2 to 5 micrometer, more preferably in the range of from 2 to 3 micrometer, and a maximum particle diameter Dv10 of at most 1.5 micrometer, preferably in the range of from 0.5 to 1.5 micrometer, more preferably from 0.5 to 1.2 micrometer.
4. The pharmaceutical composition of any of embodiments 1 to 4, comprising a pharmaceutically acceptable salt of buprenorphine selected from the group consisting of salts of buprenorphine with a carboxylic acid preferably selected from the group consisting of acetic acid, propionic acid, citric acid, tartaric acid, malic acid, maleic acid, lactic acid, malonic acid, fumaric acid, salicylic acid, succinic acid, ascorbic acid, benzoic acid, and a mixtures of two or more thereof, with a hydrohalide, preferably hydrochloride, with phosphoric acid, with sulfuric acid, and a mixture of two or more thereof, wherein the pharmaceutically acceptable salt of buprenorphine is preferably buprenorphine hydrochloride.
5. The pharmaceutical composition of any of embodiments 1 to 4, additionally comprising naloxone or a pharmaceutically acceptable salt thereof.
6. The pharmaceutical composition of embodiment 5, wherein at least 99 weight-%, preferably at least 99.5 weight-%, more preferably at least 99.9 weight-% of the naloxone or the pharmaceutically acceptable salt thereof are present in the amorphous form.
7. The pharmaceutical composition of embodiment 5 or 6, comprising a pharmaceutically acceptable salt of naloxone selected from the group consisting of salts of naloxone with a carboxylic acid preferably selected from the group consisting of acetic acid, propionic acid, citric acid, tartaric acid, malic acid, maleic acid, lactic acid, malonic acid, fumaric acid, salicylic acid, succinic acid, ascorbic acid, benzoic acid, and a mixtures of two or more thereof, with a hydrohalide, preferably hydrochloride, with phosphoric acid, with sulfuric acid, and a mixture of two or more thereof, wherein the pharmaceutically acceptable salt of naloxone is preferably naloxone hydrochloride.
8. The pharmaceutical composition of any of embodiments 5 or 7, comprising naloxone or the pharmaceutically acceptable salt thereof at a molar ratio of naloxone relative to buprenorphine in the range of from 1:1 to 5:1, preferably from 1:1 to 4:1, more preferably from 1:1 to 3:1.
9. The pharmaceutical composition of any of embodiments 1 to 8 in the form of an oral dosage composition, an injectable dosage composition, or a transdermal dosage composition, preferably an oral dosage composition, more preferably an oral film dosage composition.
10. The pharmaceutical composition of any of embodiments 1 to 9, additionally comprising at least one excipient selected from the group consisting of at least one buffer, at least one flavoring agent, at least one coloring agent, at least one plasticizer, at least one sweetener, at least one film forming agent, at least one disintegrant, at least one binder, at least one lubricant, at least one filler, at least one surfactant, at least one wetting agent, at least one coating material, and a mixture of two or more thereof.
11. The pharmaceutical composition of embodiment 10, comprising at least one buffer, optionally at least one plasticizer, and at least one film forming agent,
   wherein the ratio of the weight of the at least one buffer relative to the sum of the weights of buprenorphine or the pharmaceutically acceptable salt thereof and naloxone or the pharmaceutically acceptable salt thereof is in the range of from 0.25:1 to 0.55:1, preferably from 0.35:1 to 0.45:1;
   wherein the ratio of the weight of the at least one plasticizer, if present, relative to the sum of the weights of buprenorphine or the pharmaceutically acceptable salt thereof and naloxone or the pharmaceutically acceptable salt thereof is in the range of from 0.05:1 to 0.3:1, preferably from 0.1:1 to 0.2:1; and
   wherein the ratio of the weight of the at least one film forming agent relative to the sum of the weights of buprenorphine or the pharmaceutically acceptable salt thereof and naloxone or the pharmaceutically acceptable salt thereof is in the range of from 1:1 to 5:1, preferably from 2:1 1 to 3:1.
12. The pharmaceutical composition of embodiment 10 or 11,
   wherein the at least one buffer is selected from the group consisting of citric acid, sodium citrate, and a mixture of two or more thereof,
   wherein the at least one plasticizer, if present, is selected from the group consisting of propylene glycol, polyethylene glycol, glycerol, and a mixture of two or more thereof, and wherein the at least one film forming agent is selected from the group consisting of hydroxyethyl cellulose, hydroxypropyl cellulose, hydroxypropyl methylcellulose, and a mixture of two or more thereof.
13. The pharmaceutical composition of embodiment 12, additionally comprising at least one sweetener, at least one flavoring agent, and at least one coloring agent,
   wherein the ratio of the weight of the at least sweetener relative to the sum of the weights of buprenorphine or the pharmaceutically acceptable salt thereof and naloxone or the pharmaceutically acceptable salt thereof is in the range of from 0.4:1 to 1:1, preferably from 0.6:1 to 0.7:1;
   wherein the ratio of the weight of the at least one flavoring agent relative to the sum of the weights of buprenorphine or the pharmaceutically acceptable salt thereof and naloxone or the pharmaceutically acceptable salt thereof is in the range of from 0.001:1 to 0.01:1, preferably from 0.002:1 to 0.005:1; and
   wherein the ratio of the weight of the at least one coloring agent relative to the sum of the weights of buprenorphine or the pharmaceutically acceptable salt thereof and naloxone or the pharmaceutically acceptable salt thereof is in the range of from 0.0002:1 to 0.005:1, preferably from 0.0005:1 to 0.001:1.
14. The pharmaceutical composition of any of embodiments 10 to 13,
   wherein the at least one sweetener is selected from the group consisting of glucose (corn syrup), dextrose, invert sugar, fructose, and combinations thereof; saccharin and its various salts such as the sodium salt; dipeptide sweeteners such as aspartame, dihydrochalcone compounds, glycyrrhizin; Stevia Rebaudiana (Stevioside); chloro derivatives of sucrose such as sucralose; sugar alcohols such as sorbitol, mannitol, xylitol, and the like. Also contemplated are hydrogenated starch hydrolysates and the synthetic sweetener 3,6-dihydro-6-methyl-1-1-1,2,3-oxathiazin-4-one-2,2-dioxide, particularly the potassium salt thereof (acesulfame-K), and sodium and calcium salts thereof, natural intensive sweeteners, and a mixture of two or more thereof;
   wherein the at least one flavoring agent is selected from the group consisting of natural and synthetic flavoring liquids, such as lemon, orange, grape, lime and grapefruit and fruit essences including apple, pear, peach, grape, strawberry, raspberry, cherry, plum, pineapple, apricot or other fruit flavors, aldehydes and esters such as benzaldehyde (cherry, almond), citral i.e., alphacitral (lemon, lime), neral, i.e., beta-citral (lemon, lime), decanal (orange, lemon), aldehyde C-8 (citrus fruits), aldehyde C-9 (citrus fruits), aldehyde C- 12 (citrus fruits), to IyI aldehyde (cherry, almond), 2,6-dimethyloctanol (green fruit), and 2-dodecenal (citrus, mandarin), and a mixture of two or more thereof; and
   wherein the at least one coloring agent is selected from the group consisting of food, drug and cosmetic colors (FD&C), drug and cosmetic colors such as dyes, their corresponding lakes, natural and derived colorants, azo dyes, organic or inorganic pigments such as the oxides or iron or titanium, or coloring agents of natural origin" and a mixture of two or more thereof.
15. The pharmaceutical composition of any of embodiments 1 to 14, having a buprenorphine in vivo bioavailability ratio of at least 1.1:1, preferably at least 1.2:1, more preferably in the range of from 1.2:1 to 1.5:1, wherein the buprenorphine in vivo bioavailability ratio is defined as the buprenorphine in vivo bioavailability of the composition of any of claims 1 to 14 relative to the buprenorphine in vivo bioavailability of an otherwise identical composition comprising buprenorphine or a pharmaceutically acceptable salt thereof in the form of particles having a maximum particle diameter Dv90 of (100 ± 10) micrometer.
16. The pharmaceutical composition of any of embodiments 1 to 15 for treating, reducing, or preventing pain in a mammal, or for drug substitution therapy.
17. A process for preparing a pharmaceutical composition, preferably the pharmaceutical composition according to any of claims 1 to 16, preferably in the form of an oral dosage composition, an injectable dosage composition, or a transdermal dosage composition, more preferably an oral dosage composition, more preferably an oral film dosage composition, said process comprising
   (i) providing buprenorphine or a pharmaceutically acceptable salt thereof, preferably in the form of particles having a maximum particle diameter Dv90 of more than 20 micrometer, more preferably at least 50 micrometer, more preferably in the range of from 50 to 150 micrometer, more preferably in the range of from 75 to 125 micrometer;
   (ii) dissolving the buprenorphine or the pharmaceutically acceptable salt thereof in a solvent system obtaining a solution;
   (iii) preferably subjecting the solution obtained in (ii) to formulation, obtaining a precursor of the pharmaceutical composition comprising at least a portion of the solvent system;
   (iv) removing at least a portion, preferably at least 90 weight-%, of the solvent system comprised in the precursor of the pharmaceutical composition obtained in (iii), preferably by drying.
18. The process of embodiment 17, wherein the particles of the buprenorphine or the pharmaceutically acceptable salt thereof provided according to (i) have a maximum particle diameter Dv50 of at most 40 micrometer, preferably in the range of from 20 to 60 micrometer, more preferably in the range of from 30 to 50 micrometer, and a maximum particle diameter Dv10 of at most 10 micrometer, preferably in the range of from 6 to 10 micrometer, more preferably from 7 to 9 micrometer.
19. The process of embodiment 17 or 18, wherein the solvent system according to (ii) comprises acetone, ethanol, or a mixture thereof, and optionally additionally water, wherein the dissolving is preferably carried out at a temperature of the solvent system in the range of from 15 to 30 °C, more preferably from 18 to 25 °C, more preferably from 20 to 22 °C.
20. The process of any of embodiments 17 to 19, wherein the solution obtained in (ii) comprising the dissolved buprenorphine or the pharmaceutically acceptable salt thereof additionally comprises naloxone or a pharmaceutically acceptable salt thereof, wherein the molar ratio of the naloxone relative to the buprenorphine is preferably in the range of from 1:1 to 5:1, more preferably from 1:1 to 4:1, more preferably from 1:1 to 3:1.
21. The process of any of embodiments 17 to 20, wherein the drying according to (iv) is carried out at one or more temperatures, preferably at two or more temperatures, in the range of from 30 to 110 °C, preferably from 35 to 95 °C, more preferably from 40 to 70 °C.
22. The process of any of embodiments 17 to 21, wherein the drying according to (iv) is carried out continuously.
23. The process of any of embodiments 17 to 22, wherein the drying according to (iv) is carried out for a period of time in the range of from 3 to 30 min, preferably from 10 to 30 min, more preferably from 10 to 20 min.
24. The process of any of embodiments 17 to 23, wherein the subjecting to formulation according to (iii) comprises
   (iii.1) adding at least one excipient, preferably selected from the group consisting of at least one buffer, at least one flavoring agent, at least one coloring agent, at least one plasticizer, at least one sweetener, at least one film forming agent, at least one disintegrant, at least one binder, at least one lubricant, at least one filler, at least one surfactant, at least one wetting agent, at least one coating material, and a mixture of two or more thereof, to the solution obtained in (ii), obtaining a mixture comprising the buprenorphine or the pharmaceutically acceptable salt thereof, the at least one excipient and optionally the naloxone or the pharmaceutically acceptable salt thereof, wherein the at least one excipient is preferably added in the form of a solution or a suspension.
25. The process of embodiment 24, further comprising
   (iii.2) forming a film, preferably a wet film, from the mixture obtained in (iii.1).
26. The process of embodiment 25, wherein the film-forming is carried out at a film-forming temperature below the boiling point of the solvent system comprised in the mixture obtained in (iii.1).
27. The process of embodiment 25or 26, wherein the film-forming is carried out at a temperature of at least 18 °C, preferably at least 20 °C, preferably not more than 40°C, preferably not more than 35 °C, preferably not more than 30 °C.
28. The process of any of embodiments 25 to 27, wherein the film-forming is carried out at a temperature in the range of from 16 to 26 °C.
29. The process of any of embodiments 25 to 28, wherein the wet film obtained in (iii.2) has a thickness in the range of from 100 to 1000 micrometer.
30. The process of any of embodiments 25 to 29, wherein the dried film obtained in (iv) has a thickness in the range of from 50 to 1000 micrometer.
31. The process of any of embodiments 25 to 30, further comprising dividing the dried film obtained in (iv) into individual sub-units.
32. A pharmaceutical composition, obtainable or obtained by a process according to any of embodiments 17 to 31.
33. A pharmaceutical composition according to any of embodiments 1 to 15 or 32 for use in a method for treating, reducing, or preventing pain in a mammal or for drug substitution therapy.
34. Use of a pharmaceutical composition according to any of embodiments 1 to 15 or 32 for treating, reducing, or preventing pain in a mammal.
35. Use of a pharmaceutical composition according to any of embodiments 1 to 15 or 32 for the preparation of a medicament for treating, reducing, or preventing pain in a mammal.
36. A method for treating, reducing, or preventing pain in a mammal comprising administering a pharmaceutical composition according to any of embodiments 1 to 15 or 32 to a mammalian patient in need thereof.
37. A pharmaceutical composition according to any of embodiments 1 to 15 or 32 for use in a drug substitution therapy of a mammal, wherein the drug is preferably a narcotic.
38. Use of a pharmaceutical composition according to any of embodiments 1 to 15 or 32 in a drug substitution therapy of a mammal.
39. Use of a pharmaceutical composition according to any of embodiments 1 to 15 or 32 for the preparation of a medicament for a drug substitution therapy of a mammal.
40. A drug substitution therapy method comprising administering a pharmaceutical composition according to any of embodiments 1 to 15 or 32 to a mammalian patient in need thereof.
41. Particles of buprenorphine or a pharmaceutically acceptable salt thereof, preferably buprenorphine hydrochloride, having a maximum particle diameter Dv90 of at most 20 micrometer, preferably in the range of from 5 to 20 micrometer, more preferably from 5 to 10 micrometer.
42. The particles of embodiment 41, obtainable or obtained by a process comprising
   (Ii) providing buprenorphine or a pharmaceutically acceptable salt thereof, preferably in the form of particles having a maximum particle diameter Dv90 of more than 20 micrometer, more preferably at least 50 micrometer, more preferably in the range of from 50 to 150 micrometer, more preferably in the range of from 75 to 125 micrometer;
   (II) dissolving the buprenorphine or the pharmaceutically acceptable salt thereof in a solvent system obtaining a solution;
   (III) removing at least a portion, preferably at least 90 weight-%, of the solvent system, preferably by drying.
43. Use of the particles according to embodiment 41 or 42 for the preparation of a pharmaceutical composition, preferably an oral dosage composition, more preferably an oral film dosage composition.

The present invention is further illustrated by the following reference examples, examples, and comparative examples.

### Reference Example 1: Determination of the maximum particle diameters Dv90, Dv50, and Dv10

The maximum particle diameter Dv90 is the maximum particle diameter below which 90 % of the sample volume exists. The maximum particle diameter Dv50 is the maximum particle diameter below which 50 % of the sample volume exists. The maximum particle diameter Dv10 is the maximum particle diameter below which 10 % of the sample volume exists. The letter "D" stands for diameter, and "v" indicates that it is the volume which is used for weighting the distribution of the particles.

According to the present invention, the Dv90, Dv50 and Dv10 of the buprenorphine particles contained in a pharmaceutical composition were determined according to the following method:

### Preparation of a saturated buprenorphine hydrochloride solution

4.1 g buprenorphine hydrochloride were filled in a 500 ml HPLC bottle. 250 ml UHQ water were added, and the resulting suspension was put for 15 minutes into an ultrasonic bath. Occasionally, the suspension was shaken. A magnetic stir bar was put into the suspension, and the suspension was heavily stirred for 2 hours on a magnetic stirrer, and occasionally floating particles were mixed into the water. Then, the suspension was left for complete saturation for two days. The obtained solution was not clear, and in the solution, some crystals were visible. Therefore, the solution is saturated.

The resulting suspension was filtered through a Buechner funnel, wherein the filtrate was the saturated aqueous buprenorphine hydrochloride solution. This saturated aqueous buprenorphine hydrochloride solution was clear and used further immediately.

### Sample preparation

One or more pharmaceutical compositions comprising buprenorphine hydrochloride, with a total weight of the buprenorphine hydrochloride of about 16 g were given into a test tube. 5 ml of the saturated aqueous buprenorphine hydrochloride solution prepared according to section 1. above were added and well-mixed on a Vortexer. Afterwards, the homogeneously dispersed sample was filled with a pipette into the measurement cell of a Mastersizer 2000 to achieve a measurable signal in the Mastersizer 2000 with an obscuration of from 10 to 25%. (If the buprenorphine hydrochloride particles in the pharmaceutical composition turns out to be very coarse, the amount of suspension may not be sufficient for achieving the specified obscuration; in this case, a higher amount of pharmaceutical composition has to be processed.)

### Measuring process

Prior to the measurement, it must be verified that the measurement cell is exclusively filled with saturated aqueous buprenorphine hydrochloride solution since residual water would lead to dissolution of sample particles. Thus, prior to the measurement, the measurement cell was washed twice with saturated aqueous buprenorphine hydrochloride solution.

Afterwards, the measurement cell was filled with 80 ml of the saturated aqueous buprenorphine hydrochloride solution, and an optical alignment of the laser and a background measurement were run, the result having being automatically subtracted. The cell was filled with the amount of dispersed sample obtained according to section 2. above corresponding to the required concentration according to an obscuration in the range of from 10 to 25 %. Immediately after the dispersion was stable, the particle size distribution measurement was started.

The measurement is performed with the following: instrument settings:
Dispersant refractive index: 1.33

### Measurement model: Fraunhofer / general purpose

A measurement sequence consisted of 8 single measurements, and the respective mean value was represented in a histogram report (distribution curve and table of measured values). The histogram showed the particle size distribution of the buprenorphine hydrochloride in the pharmaceutical composition, whereas all other excipients were dissolved in the aqueous saturated buprenorphine hydrochloride solution.

### Reference Example 2: Determination of in vivo bioavailability of buprenorphine

The *in vivo* bioavailability of buprenorphine was determined according to Guideline on Bioavailability and Bioequivalence Studies for Orally Administered Drug Product, issued by U.S. Department of Health and Human Services, Food and Drug Administration, Center for Drug Evaluation and Research (CDER) in March 2003. The following parameters were determined in the blood samples of healthy individuals:
- AUC_{inf}: the area under the plasma concentration curve extrapolated to infinite time
- AUCₜ: the area under the plasma concentration curve from administration to last observed concentration at time t
- cₘₐₓ: the maximum plasma concentration
- tₘₐₓ: the time to maximum plasma concentration

### Example 1: Preparation and testing of an oral film dosage composition comprising buprenorphine hydrochloride particles having a maximum particle diameter Dv90 of 7 micrometer

### E1.1 Preparation of the oral film dosage composition

### Preparation of the Mix (coating solution)

Buprenorphine HCl was weighed into a weighing pan and transferred into the preparation vessel 1. Then, Naloxone HCl 2H₂O was weighed into a weighing pan and transferred into the preparation vessel 1. Lemon oil USP was weighed into a weighing pan (50 mL glass vessel) and transferred into the preparation vessel 1. Acetone Ph.Eur/USP was added into the preparation vessel 1. Tri-Sodium citrate anhydrous USP, Citric acid monohydrate Ph.Eur, USP, Acesulfam K Ph.Eur, USP/NF, Maltitol solution USP/NF were weighed into the preparation vessel 2. Dualcert FD&C Yellow 6 was added into a weighing pan and transferred into the preparation vessel 2. Purified water was added into the preparation vessel 2. The excipient solution in preparation vessel 2 was transferred into the preparation vessel 1. Polyox WSR N10 NF, Polyox WSR N80 NF, Polyox WSR 1105 NF and Pharmacoat 606 PhEur. USP/NF were weighed into a sufficient weighing pan and sprinkled the substance onto the contents of the preparation vessel 1 while stirring. The content of the preparation vessel 1 was stirred by a paddle stirrer until all components have been dissolved, but for min. 15 minutes. The mix was stirred for at least 12 hours at approximately 50 RPM (rounds per minute) until it was further processed.

### Coating and drying of the laminate

| | | |
|---|---|---|
| Coating technology: | | Knife coating |
| Coating speed: | | 0.5 m/min |
| | | |
| Drying technology: | | 3 Hot air dryers |
| | Dryer 1: | length 1.5 m / temperature setting 45°C / time in Dryer 1: 3 minutes |
| | Dryer 2: | length 1.5 m / temperature setting 55°C / time in Dryer 2: 3 minutes |
| | Dryer 3: | length 2.5 m / temperature setting 65°C / time in Dryer 3: 5 minutes |
| Inlet Air temperature: | | 21°C |
| Inlet Air humidity: | | 4.5g/kg absolute |

Coat weight after drying : 168.45 g/ m².

The dry product contained residual solvents of app. 50000 ppm acetone and 3-6 weight-% water. A coating machine from Company Mathis AG of Switzerland was used.

The obtained oral film dosage composition had the following composition:

| **component** | | **amount / mg** |
|---|---|---|
| | | |
| buprenorphine hydrochloride | Dv90 = 7 micrometer | 8.63 |
| | Dv50 = 2 micrometer | |
| | Dv10 = 1 micrometer | |
| naloxone hydrochloride | | 2.44 |
| trisodium citrate (anhydrous) | | 1.34 |
| citric acid monohydrate | | 2.95 |
| acesulfame K | | 1.50 |
| malitol solution / NF | | 6.01 |
| polyethylene oxide 100000 (Mw) | | 6.01 |
| polyethylene oxide 200000 (Mw) | | 13.52 |
| polyethylene oxide 900000 (Mw) | | 2.41 |
| Pharmacoat 606 (hypromellose) | | 2.11 |
| lemon oil according to USP monograph | | 0.05 |
| coloring agent FD&C yellow no. 6 (sunset yellow) | | 0.01 |
| | | |
| size / cm² | | 2.78 |

### E1.2 Testing of the oral film dosage composition according to Reference Example 2

The study was an open-label, single-dose, randomized, three-period, two-treatment, three-sequence, crossover, partial replicate, comparative bioavailability study in healthy male and female subjects under fasting conditions. Blood samples were collected prior to drug administration up to 120 hours post dose (25 time points). Buprenorphine, norbuprenorphine, unconjugated naloxone and total naloxone concentrations were measured from plasma by validated LC/MS/MS analytical method. ANOVA was performed on the log-transformed (natural logarithm) of AUCt, AUCinf, and Cmax parameters using PROC GLM in SAS® for all analytes. For each pharmacokinetic parameter, estimates were obtained for the treatment mean values, µT and µR, for the difference between the treatment means with the corresponding 90% confidence interval, and for the within-subject variance for the reference treatment.

Buprenorphine of the Example 1 had the following *in vivo* availability: 90% confidence interval (CI) of AUCₜ was 119-131 %, AUC_{inf} 119-130 % and cₘₐₓ 120-138 %.

### Comparative Example 1: Preparation and testing of an oral film dosage composition comprising buprenorphine hydrochloride particles having a maximum particle diameter Dv90 of 90 micrometer

### CE1.1 Preparation of the oral film dosage composition

### Preparation of the Mix (coating solution)

The mix was prepared into a MasterPlant200 Mixer from the company IKA. This device has two RFGC Mixers (Comparable to paddle stirrers) and one DBI mixer (high sheer mixer comparable to an ultra turrax). Water was added into the Mixer and the mixing speed was set to RFGC-1 to 85 RPM and RFGC-0 to 30 RPM. The mixture was mixed for 5 minutes. Lemon oil, Maltitol solution USP/NF, tri-Sodium citrate anhydrous USP, Citric acid monohydrate Ph.Eur ,USP, Acesulfam K Ph.Eur, and Dualcert FD&C Yellow 6 and were added it into the MP200 and mixed for 10 minutes at RFGC-1 set to 85 RPM and RFGC-0 set to 30 RPM. Buprenorphine HCl and Naloxone HCl 2H₂O were weighed and transferred into the mixer and the ingredients were mixed for 10 minutes at RFGC-1 set to 85 RPM and RFGC-0 set to 30 RPM. High sheer mixer was then set to 2000 RPM and the mixture was mixed for 30 minutes. Polyox WSR N10 NF, Polyox WSR N80 NF, Polyox WSR 1105 NF and Methocel E5 (comparable HPMC to Pharmacoat 606 PhEur. USP/NF) were added into the Mixer and mixed for 10 minutes at RFGC-1 set to 85 RPM and RFGC-0 set to 30 RPM. High sheer mixer was set to 2000 RPM and the mixture was mixed for 60 minutes.

### Coating and drying of the laminate

| | | |
|---|---|---|
| Coating technology: | | slot die coating |
| Coating speed: | | 0.6 m/min |
| | | |
| Drying technology: | | 4 Hot air dryers |
| | Dryer 1: | length 2.5 m / temperature setting 90°C / time in Dryer 1: 4.2 minutes |
| | Dryer 2: | length 2.5 m / temperature setting 90°C / time in Dryer 2: 4.2 minutes |
| | Dryer 3: | length 2.5 m / temperature setting 90°C / time in Dryer 3: 4.2 minutes |
| | Dryer 4: | length 2.5 m / temperature setting 90°C / time in Dryer 4: 4.2 minutes |
| Inlet Air temperature: | | Room temperature |
| Inlet Air humidity: | | not defined |

Target coat weight after drying :160 g/m². The product contained residual solvent water of approximately 3-6 weight-%.
Target dry coat weight: 159.33 g/ m². A coating machine S-coater from the manufacturer Frontier was used.

The obtained oral film dosage composition had the following composition:

| **component** | | **amount / mg** |
|---|---|---|
| | | |
| buprenorphine hydrochloride | Dv90 = 90 micrometer | 8.63 |
| | Dv50 = 30 micrometer | |
| | Dv10 = 6 micrometer | |
| naloxone hydrochloride | | 2.44 |
| trisodium citrate (anhydrous) | | 1.34 |
| citric acid monohydrate | | 2.95 |
| acesulfame K | | 1.50 |
| malitol solution / NF | | 6.01 |
| polyethylene oxide 100000 (Mw) | | 6.01 |
| polyethylene oxide 200000 (Mw) | | 13.52 |
| polyethylene oxide 900000 (Mw) | | 2.41 |
| Pharmacoat 606 (hypromellose) | | 1.05 |
| lemon oil according to USP monograph | | 0.05 |
| coloring agent FD&C yellow no. 6 (sunset yellow) | | 0.01 |
| | | |
| size / cm² | | 2.78 |

### CE1.2 Testing of the oral film dosage composition according to Reference Example 2

The study was an open-label, single-dose, randomized, two-period, two treatment, two-sequence, crossover, comparative bioavailability pilot study in healthy male and female subjects under fasting conditions. Blood samples were collected prior to drug administration up to 72 hours post dose (25 time points). Buprenorphine, norbuprenorphine, unconjugated naloxone and total naloxone concentrations were measured from plasma by validated LC/MS/MS analytical method. Pharmacokinetic parameters were estimated for all analytes using a non-compartmental approach. ANOVA (PROC GLM) was performed on log-transformed AUCt, AUCinf and Cmax and untransformed Tmax, Kel and Thalf. Based on log-transformed data, ratios of the geometric means for treatments and the corresponding 90% confidence intervals were calculated for AUCt, AUCinf and Cmax.

Buprenorphine of the Comparative Example 1 had the following *in vivo* availability: 90% CI of AUCₜ was 97-117 %, AUC_{inf}96-114 % and cₘₐₓ 101-126 %.

### Comparative Example 2: Commercial Product

The commercial product had the following composition:

| **component** | | **amount / mg** |
|---|---|---|
| | | |
| buprenorphine hydrochloride | Dv90 = 100 micrometer | 8.63 |
| | Dv50 = 30 micrometer | |
| | Dv10 = 5 micrometer | |
| naloxone hydrochloride | | 2.44 |
| trisodium citrate (anhydrous), citric acid monohydrate, acesulfame K, malitol solution / NF, polyethylene oxide 100000, polyethylene oxide 200000, polyethylene oxide 900000, Pharmacoat 606, lemon aroma, coloring agent FD&C yellow no. 6 | | |

### Literature cited

- WO 2011/017483
- Guideline on Bioavailability and Bioequivalence Studies for Orally Administered Drug Product, U.S. Department of Health and Human Services, Food and Drug Administration, Center for Drug Evaluation and Research (CDER), March 2003, BP, Revision 1

## Claims

1. A pharmaceutical composition comprising buprenorphine or a pharmaceutically acceptable salt thereof in the form of particles having a maximum particle diameter Dv90 of at most 20 micrometer.

2. The pharmaceutical composition of claim 1, wherein the Dv90 is in the range of from 5 to 20 micrometer, preferably from 5 to 10 micrometer.

3. The pharmaceutical composition of any of claims 1 or 2, comprising a pharmaceutically acceptable salt of buprenorphine selected from the group consisting of salts of buprenorphine with a carboxylic acid preferably selected from the group consisting of acetic acid, propionic acid, citric acid, tartaric acid, malic acid, maleic acid, lactic acid, malonic acid, fumaric acid, salicylic acid, succinic acid, ascorbic acid, benzoic acid, and a mixtures of two or more thereof, with a hydrohalide, preferably hydrochloride, with phosphoric acid, with sulfuric acid, and a mixture of two or more thereof, wherein the pharmaceutically acceptable salt of buprenorphine is preferably buprenorphine hydrochloride.

4. The pharmaceutical composition of any of claims 1 to 3, additionally comprising naloxone or a pharmaceutically acceptable salt thereof.

5. The pharmaceutical composition of any of claims 1 to 4 in the form of an oral dosage composition, an injectable dosage composition, or a transdermal dosage composition, preferably an oral dosage composition, more preferably an oral film dosage composition.

6. The pharmaceutical composition of any of claims 1 to 5, additionally comprising at least one excipient selected from the group consisting of at least one buffer, at least one flavoring agent, at least one coloring agent, at least one plasticizer, at least one sweetener, at least one film forming agent, at least one disintegrant, at least one binder, at least one lubricant, at least one filler, at least one surfactant, at least one wetting agent, at least one coating material, and a mixture of two or more thereof.

7. The pharmaceutical composition of any of claims 1 to 6, having a buprenorphine in vivo bioavailability ratio of at least 1.1:1, preferably at least 1.2:1, more preferably in the range of from 1.2:1 to 1.5:1, wherein the buprenorphine in vivo bioavailability ratio is defined as the buprenorphine in vivo bioavailability of the composition of any of claims 1 to 6 relative to the buprenorphine in vivo bioavailability of an otherwise identical composition comprising buprenorphine or a pharmaceutically acceptable salt thereof in the form of particles having a maximum particle diameter Dv90 of (100 ± 10) micrometer.

8. A process for preparing a pharmaceutical composition, preferably the pharmaceutical composition according to any of claims 1 to 7, preferably in the form of an oral dosage composition, an injectable dosage composition, or a transdermal dosage composition, more preferably an oral dosage composition, more preferably an oral film dosage composition, said process comprising
(i) providing buprenorphine or a pharmaceutically acceptable salt thereof, preferably in the form of particles having a maximum particle diameter Dv90 of more than 20 micrometer, more preferably at least 50 micrometer, more preferably in the range of from 50 to 150 micrometer, more preferably in the range of from 75 to 125 micrometer;
(ii) dissolving the buprenorphine or the pharmaceutically acceptable salt thereof in a solvent system obtaining a solution;
(iii) preferably subjecting the solution obtained in (ii) to formulation, obtaining a precursor of the pharmaceutical composition comprising at least a portion of the solvent system, the subjecting to formulation preferably comprising
(iii.1) adding at least one excipient to the solution obtained in (ii), obtaining a mixture comprising the buprenorphine or the pharmaceutically acceptable salt thereof, the at least one excipient and optionally the naloxone or the pharmaceutically acceptable salt thereof;
(iii.2) forming a film, preferably a wet film, from the mixture obtained in (iii.1);
(iv) removing at least a portion, preferably at least 90 weight-%, of the solvent system comprised in the precursor of the pharmaceutical composition obtained in (iii), preferably by drying.

9. The process of claim 8, wherein the solvent system according to (ii) comprises acetone, ethanol, or a mixture thereof, and optionally additionally water, wherein the dissolving is preferably carried out at a temperature of the solvent system in the range of from 15 to 30 °C, more preferably from 18 to 25 °C, more preferably from 20 to 22 °C.

10. The process of claim 8 or 9, wherein the solution obtained in (ii) comprising the dissolved buprenorphine or the pharmaceutically acceptable salt thereof additionally comprises naloxone or a pharmaceutically acceptable salt thereof, wherein the molar ratio of the naloxone relative to the buprenorphine is preferably in the range of from 1:1 to 5:1, more preferably from 1:1 to 4:1, more preferably from 1:1 to 3:1.

11. A pharmaceutical composition, obtainable or obtained by a process according to any of claims 8 to 10.

12. A pharmaceutical composition according to any of claims 1 to 7 or 11 for use in a method for treating, reducing, or preventing pain in a mammal or for drug substitution therapy.

13. Particles of buprenorphine or a pharmaceutically acceptable salt thereof, preferably buprenorphine hydrochloride, having a maximum particle diameter Dv90 of at most 20 micrometer, preferably in the range of from 5 to 20 micrometer, more preferably from 5 to 10 micrometer.

14. The particles of claim 13, obtainable or obtained by a process comprising
(I) providing buprenorphine or a pharmaceutically acceptable salt thereof, preferably in the form of particles having a maximum particle diameter Dv90 of more than 20 micrometer, more preferably at least 50 micrometer, more preferably in the range of from 50 to 150 micrometer, more preferably in the range of from 75 to 125 micrometer;
(II) dissolving the buprenorphine or the pharmaceutically acceptable salt thereof in a solvent system obtaining a solution;
(III) removing at least a portion, preferably at least 90 weight-%, of the solvent system, preferably by drying.

15. Use of the particles according to claim 13 or 14 for the preparation of a pharmaceutical composition, preferably an oral dosage composition, more preferably an oral film dosage composition.
